# EUROPEAN PATENT SPECIFICATION

(11) **EP 2 979 730 B1**
(45) Date of publication and mention of the grant of the patent: **25.04.2018**
(21) Application number: 15178487.3
(22) Date of filing: 27.07.2015
(51) Int. Cl.: A61Q 1/02, A61Q 1/12, A61K 8/02, A45D 33/18, B30B 15/02, B30B 11/22

(54) **MAKE-UP ELEMENT, AND METHOD TO PRODUCE IT**
MAKE-UP-ELEMENT UND VERFAHREN ZUR HERSTELLUNG DAVON
ÉLÉMENT DE MAQUILLAGE ET PROCÉDÉ POUR PRODUIRE CELUI-CI

(30) Priority: 31.07.2014 IT MI20141408; 26.11.2014 IT MI20142037
(43) Date of publication of application: 03.02.2016
(73) Proprietor: Chromavis S.p.A., 20122 Milano (IT)
(72) Inventor: BRAMBILLA, Andreina, 20060 Pozzuolo Martesana (MI) (IT); DONIDA, Giulia Maria, 26015 Soresina (CR) (IT)
(74) Representative: Vanosi, Adelio Valeriano

(56) References cited:
- JP-A- H0 482 818
- JP-A- S62 145 008
- JP-A- 2000 202 272
- JP-A- 2013 063 934
- KR-A- 20120 019 151
- US-A- 3 800 034
- US-A- 3 972 666

## Description

The present innovation relates to a make-up element and to a method for producing it.

The invention also relates to a cosmetic product for make-up produced by using the make-up element.

Various make-up elements, make-up products and methods of their production are known, see for example US 3 972 666, JP 2013 063934 and JP H04 82818. Make-up products (known as "beads powders") are known on the market, produced by combining a plurality of make-up beads equal to one another, or of different colors, placed in a container. The beads crumble under the action of a brush, on which they transfer a part of the powder which compose them. The brush is then used to make up face, eyes and body.

The beads consist of a make-up powder and their production method, which consists in the coating of a waxy material core through the use of a coating pan, with multi-layers of powder bound thanks to sugar aqueous solutions, is quite complicated and quite slow. In fact, the beads, once formed, must be dried in an oven to evaporate the volatile component required for their formation. Moreover, the current production method dictates that each bead has a surface make-up bead of a single color.

EP2106782-A1 describes a method to implement a plurality of multi-layer cosmetic powder beads inside a rotating drum mixer. The beads are placed inside of a bottom and are slightly pressed. Then, a surface layer is removed from the beads themselves in order to highlight the different colors and a further pressing is carried out.

The final effect is to obtain a cosmetic product which has a matrix in which more or less large intact beads are scattered; the closest ones to the surface of the cosmetic product are cut to highlight the different colored circular coatings of which they are formed.

The object of the present invention is to provide a less expensive and easier to produce alternative to the traditional make-up beads, while ensuring the same cosmetic benefits.

Another object of the present invention is to provide a multicolor cosmetic product for make-up with an aesthetic effect and which is easy, inexpensive and quick to produce.

This and other objects are achieved by a make-up product and by a make-up element produced according to the technical teachings of the appended claims.

Advantageously, the cosmetic product for make-up or the make-up element according to the present invention makes make-up powders of different color immediately available on its surface.

Further features and advantages of the invention will become apparent from the description of a preferred but non-exclusive embodiment of the invention, shown by way of a non-limiting example in the accompanying drawings, in which:
figure 1 is a simplified sectional view of a product for make-up;
figure 2A is an axial sectional view of a make-up element part of the product for make-up in figure 1;
figure 2B is a plan view of the make-up element in figure 2A;
figure 3 is a simplified section of an equipment for producing a plurality of make-up elements such as those in figure 2;
figure 4 is a simplified section of a different equipment for producing the make-up elements in figure 2A, 2B;
figure 5 is yet another simplified section of a different equipment for producing the make-up elements in figure 2A, 2B;
figure 6 is a side view of a different embodiment of a make-up element;
figures 7, 8 diagrammatically show subsequent steps of production of the cosmetic product for make-up according to the present invention;
figure 9 shows an optional working, subsequent to those of the preceding figures;
figure 10 is a simplified side sectional view of the cosmetic product for make-up of the present description;
figure 11 is a top plan view of the cosmetic product for make-up in figure 10.

With reference to the above figures, a make-up element is shown, indicated with reference numeral 1.

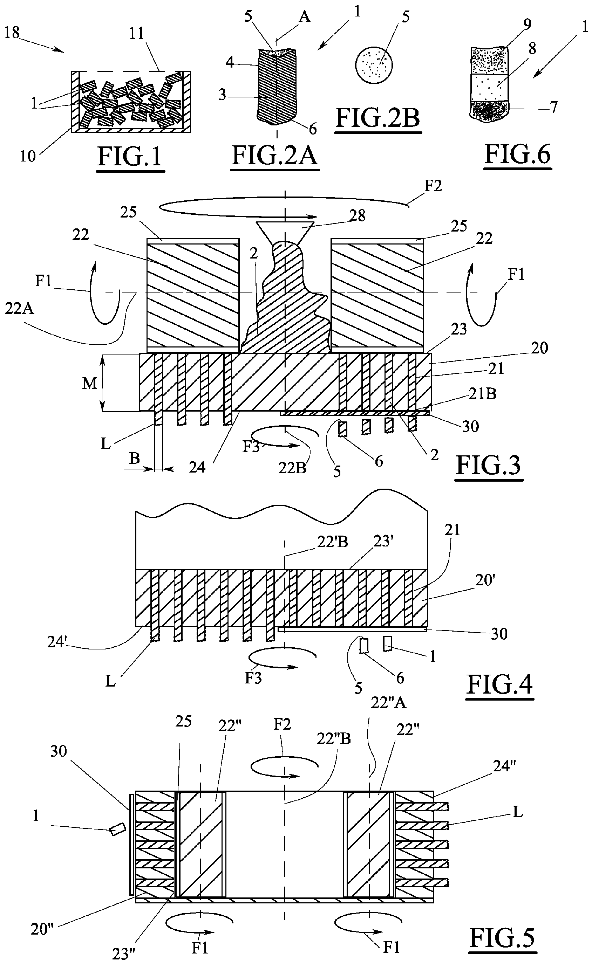

The make-up element 1, shown in detail in figures 2A and 2B, comprising a body 3 formed of a pressed make-up powder 2. The body is basically shaped as a prism with mainly axial development along the longitudinal axis A. In the embodiment shown herein, the prism has a circular section (see figure 2B), but obviously the shape of the section may be any, such as elliptical, square, rectangular, polygonal, star, etc.

In the present text, "prism" means an elongated element with a mainly axial development, having the same cross section for most of its length.

In the practice, the cross section of the prism is not the same only in proximity of its bases, which may have, as already said, a circular (see figure 2B), elliptical, square, rectangular, polygonal heart, star shape, etc.

The body includes a lateral wall 4, which is defined and regular. In the present text, the term 'defined and regular' surface means that it does not have irregularities in shape, even if it has an inherent surface roughness since the surface precisely consists of a pressed powder.

In the example described, therefore, where the cross section of the element is circular, the defined and regular surface is the lateral one of a cylinder. Depending on the shape of the base, it may consist of more parallelograms placed side by side.

The make-up element is then provided with a first 5 and a second base 6 which, unlike the lateral surface 4, have an irregular surface.

In the present text, 'irregular surface' means that it is not specifically defined, particularly as it results from a breaking operation in a predefined area. The breaking occurs substantially along a random tear line, of a strip L from which the make-up element is formed. Precisely by virtue of the methods of producing the piece, which will be described hereafter, the first base 5 has a concave shape, while the second base 6 has a convex shape.

Advantageously, the body 3 may have a maximum width of between 2 and 10mm, preferably 6mm, and a length is between 2 and 15mm, preferably between 4 and 15mm, and even more preferably 8mm.

In the example in figures 2A and 2B, the make-up element is made of a single make-up powder, while in the example in figure 6, the make-up element is multilayer and has three different colors, since it consists of three different make-up powders.

As can be seen, a first layer 9 of pressed make-up powder is superimposed in the axial direction to a second layer 8 of pressed make-up powder of a different color. In turn, the second layer can be superimposed on to a third layer 7 and so on, up to obtain a make-up element with a maximum of 5 layers of different color.

In figure 1 it may be seen that the various make-up elements are arranged inside a container 10, which can be provided with a cover 11, to form a product for make-up 18. The different make-up elements are randomly arranged within the container and may have all the same color, or they may have colors different from each other.

The method for the production of the make-up elements 1 described above can provide for mixing with each other, preferably through a mill, at least some pigments, a filler and a binder to obtain a make-up powder.

The pigments/beads can be included in a weight percentage from 5 to 43% and may also include beads or glitters (polyethylene terephthalate) in a maximum percentage of 10%. For example, the pigments/beads can be selected from those listed hereafter: mica, calcium sodium borosilicate, silica, synthetic fluorphlogopite, calcium aluminum borosilicate, ci 77891 (titanium dioxide), ci 77491 (iron oxides), ci 77288 (chromium oxide greens), ci 75470 (carmine), ci42090 (blue 1 lake), ci 77289 (chromium hydroxide green), ci 77510 (ferric ammonium ferrocyanide), ci 77742 (manganese violet), ci 45410 (red 28 lake), ci 15850, (red 7), ci 77007 (ultramarines), ci 19140 (yellow 5 lake), ci77163 (bismuth oxychloride), ci 77499 (iron oxides), ci 77510 (ferric ferrocyanide), ci 77492 (iron oxides).

The binders may instead be included in a percentage from 5 to 10% by weight, and as an example they may be selected from one or more of those belonging to the following group: esters (pentaerythrityl tetraisostearate, diisostearyl malate, octyldodecyl stearoyl stearate, isopropyl isostearate, isosterayl neopentanoate...), triglycerides (triethylheaxanoin, gliceryl stearate, gliceryl caprilate,...), silicone oils (dimethicone), butters (theobroma cacao butter, butyrospermum parkii ...), waxes (both of natural and synthetic origin).

As regards the fillers, instead, they may be provided in a percentage from 13 to 90%.

They may for example be selected from one or more of those belonging to the following group: talc, magnesium myristate, zinc stearate, starch, kaolin.

The make-up powder 2 may also include one or more preservatives, in a weight percentage from 0.3 to 1%, and one or more perfumes, in a percentage from 0.1 to 1%.

To improve the flowability of the powder, both during the formation of the make-up elements 1 and during their use, it is possible to provide for the presence of flowing agents in a weight percentage from 0.5 to 1.5%. They are for example selected from one or more elements of those belonging to the following group: polymethyl methacrylate, polyurethane, boron nitride, nylon 12, etc.

The make-up powder 2, produced as described above, is pressed against a matrix 20 provided with a plurality of holes 21, of a shape corresponding to the cross section of the make-up element to be obtained. Thus, to get a make-up element of circular cross section, such as that shown above, holes 21 of matrix 20 will be through and circular. The powder can be pressed into the holes of the matrix by means of a machine of conception similar to a pellet mill, as that diagrammatically shown in figure 3.

In particular, with reference to the figure cited above, the make-up powder 2 is fed in any known manner, and for example by a screw feeder which empties into a hopper 28, between two rotating rolls 22 above matrix 20. The rolls, besides rotating about their own axis 22A positioned horizontally (arrow F1), also rotate about a common vertical axis 22B (arrow F2). It is to be noted that at their outer surfaces, the rolls are provided with preferably axial grooves 25. Alternatively or in addition to grooves 25, the rolls may be superficially provided with blind holes.

During their movement, rolls 22 trap the powder into grooves 25 and press it (through their surface not affected by the grooves) on a first flat surface 23 of matrix 20, conveying the make-up powder within a first end 21A of holes 21.

The operation generates heat which is transferred to the make-up powder, heating it and mixing the binder with the other components. It was verified that the temperature which is reached at steady state in the pressing step is between 30° and 70 °C. It should be said that the percentage moisture of the powder is very important in the process, and by way of example it is from 0.8 to 1.5%.

Pushing the make-up powder 2 in holes 21, a plurality of strips L are formed which protrude from a second end of holes 21B placed on a second surface 24 of matrix 20.

Underneath such a second surface, a blade movable with respect to the second surface 24 simultaneously breaks multiple strips so as to form a plurality of make-up elements.

Blade 30, for example, breaks the strips protruding from the second end of the holes by a length of between 2 and 15mm, preferably between 4 and 15mm, more preferably 8mm.

Furthermore, the holes of the matrix may have a maximum diameter of between 2 and 10mm, preferably 6mm.

For the operation of 'pelletization' of the make-up powder to take place correctly, the ratio between the hole diameter and height M of the matrix is important. To obtain a proper formation of strips L first, and of the make-up elements after, the ratio B/M must be between 0.16 and 0.22 (diameter B of the holes is between 2 and 10mm, while the height of the matrix ranges from 12 to 45mm). It may in particular be such as to generate a pressure, in the axial position of the holes, between 20 and 100 bar.

It should be noted that since strips L are made by pressing the make-up powder inside holes 21 and breaking strips L as described, it results that one of the bases of the make-up element has a concave shape, while the other has a convex shape.

In fact, the pressure exerted by the rolls is distributed on the powder inside the holes, creating more compressed areas along the perimeter of each hole (and thus along the perimeter surface of the make-up element) and less compressed areas in the vicinity of the hole axis. This pressure distribution in each strip L and in each make-up element imposes preferential rupture surfaces of substantially curved shape. In fact, when a strip is broken, the part of strip still protruding from the matrix has a convexity, while the make-up element has a concavity corresponding to the convexity of the strip. Therefore, each make-up element thus produced will have a first base 5 with concave surface, and a second base 6 with convex surface.

Since each make-up element 1 is realized with the above-described method, it will have, as already mentioned, a lateral surface defined and regular, where the only imperfections of shape will be due to the particle size and porosity of the powder with which the element is formed, and to axial streaks due to the flow of the powder in the holes of the matrix. On the other hand, the bases will have an irregular surface resulting from a random break of each strip.

It should be noted that the rolls as represented have an axis parallel to a first surface 23 of the matrix. However, it is also possible to provide one or more rolls with an axis of rotation perpendicular to such a surface 23. In this case, the rolls will press the powder with their circular surface facing the matrix.

The embodiment in figure 4, in which to identify parts similar to those already described, the same reference numerals already mentioned above will be used, with an index added, shows a matrix 20' similar to that already described. It differs from the previous one for the presence of holes 21 also at a portion thereof close to axis 22'B. The make-up powder is fed on the first surface already in pressure, for example by means of the screw of an extruder (not shown). The operation of this embodiment is substantially similar to that already described, and does not require further description.

The embodiment of figure 5 instead is provided with a matrix 20" of annular shape, and with rolls with a rolling axis perpendicular to a first cylindrical surface 23" of the matrix. The axes of rotation of the rolls also rotate about a central axis 23"B. Also in this case, the operation is obvious to a man skilled in the art. The only difference with the previous embodiments is that the make-up elements are formed laterally to the matrix, and not frontally as in the previous cases.

It should be noted that multicolor make-up elements such as that shown in figure 6 can easily be produced by feeding the matrix (however made) with powders of different color in a precise sequence and in precise amounts.

The make-up elements 1 manufactured as described above can be directly used for make-up, packaged for example as shown in figure 1, or they may be further processed to obtain a cosmetic product 50 for make-up and preferably multicolored.

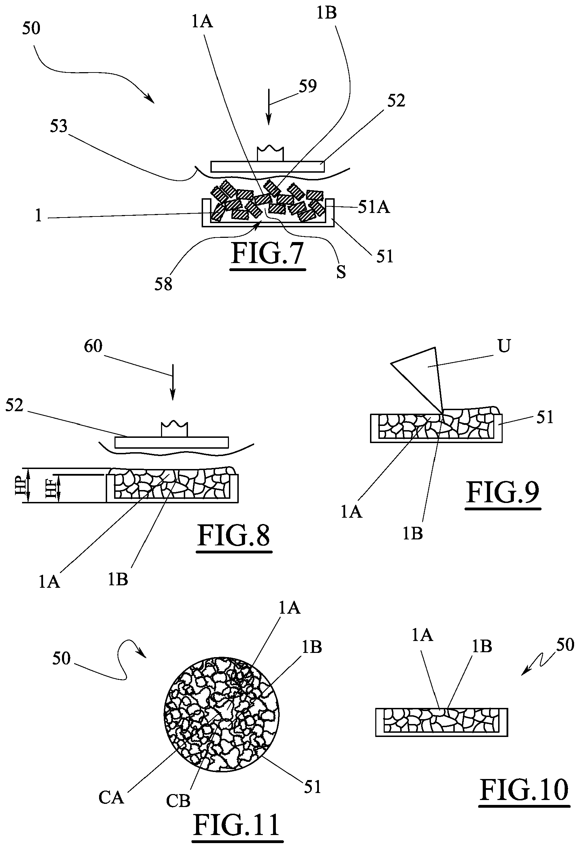

The various processing steps starting from make-up elements produced as described above (in this case actual pellets) are shown in figures 7-9.

In a first step (fig. 7), the make-up elements are positioned on a bottom 51 (or base). Bottom 51 is advantageously of the shaped type and has a lateral wall 51A adapted to contain laterally the make-up elements 1 positioned therein. In fact, the bottom wall and the lateral one of the bottom define a delimited volume 58 within which the make-up elements 1 are arranged.

It should be said that the delimited volume can also be made in different ways. For example, it is possible to provide a flat bottom above which a cylindrical annular body is temporarily positioned in contact, which acts as a lateral containment wall for the make-up elements.

The make-up elements 1 placed above the bottom are preferably of at least two different colors so as to obtain, after processing, a multicolor cosmetic product 50. However, it possible to envisage the use of make-up elements with different length, diameter or even shape (round, square, heart, etc...) to obtain particular aesthetic effects.

For example, some make-up elements of diameter 2mm and length 3mm, other of diameter 4 mm and length 6 mm, still others with different shapes and cross sections, and so on, may be arranged above the bottom.

It should be said, moreover, that it is possible to simultaneously use cosmetic elements which consist of a cosmetic powder which has different percentages of binder.

For example, cosmetic elements may be used, made with a powder very rich in binder along with others made with a cosmetic powder less rich in binder, which will be more easily crushed than the first ones.

A further step of the process involves compressing the make-up elements 1 through a mold 52 suitably operated. The compression is advantageously carried out by interposing a canvas 53 between the make-up elements themselves 1 and mold 52.

Mold 52 acts on the make-up elements (arrow 59) with such a pressure as to deform and/or break the make-up elements 1, pushing them to occupy the entire volume between the bottom and the mold.

For example, the pressure is in the range between 20 and 60 bar, preferably 40 bar.

Thereby, the cosmetic powder which is separated from the crushed and/or deformed make-up elements fills the gaps S present between the various make-up elements in the first step, when they are simply lying (arranged randomly) inside the delimited volume 58.

The operation allows obtaining a cosmetic product for make-up with substantially uniform density, and free of voids or residual air.

As can be seen in figure 8, the height HF of the lateral containment wall 51A of the bottom may be less than the height of the cosmetic HP at the end of the pressing. In the practice, it is advantageous to leave an excess of cosmetic material after the compacting step which protrudes above the lateral wall of bottom 51.

At the end of the compacting step, mold 52 is lifted (figure 8, arrow 60) and the surface of the cosmetic is worked with a suitable tool U, which may be for example a milling cutter, a sponge or another abrasive means. It is also possible that the surface of the cosmetic be turned.

Thereby, the superficial part of the semi-finished product of which the final cosmetic product will be made is removed.

At the end of the above-described processing, the multicolor cosmetic which is obtained is that shown in figure 11. It has a multicolored spotted surface which optionally can undergo a further compacting step.

The further compacting step may take place with the same mold 52, with a smooth surface, or with another mold which creates grooves on the surface of the cosmetic product (for example, decorations, or a trademark).

Such a surface, by virtue of the method used to produce the cosmetic product, has various areas of irregular shape and contour CA, CB and possibly of a different color, mutually placed side by side. Each area is originated from the deformation or crushing (or both) of a make-up element 1A, 1B.

If multiple make-up elements of the same color are adjacent during crushing/deformation, a larger area of a single color will be created. Even within a region of a same color, however, it will be possible to distinguish visually (for example by tilting the cosmetic product in order to observe the cut surface), the contour CA, CB of each individual make-up element crushed, at least partially deformed and/or crushed by compression. It is noted in particular that, upon the compression, in the example exerted by mold 52, all the make-up elements originally present on the bottom are either crushed or deformed or both crushed and deformed.

It should be noted that the use of a plurality of make-up elements of make-up powder in agglomerated form, either deformed or crushed, to obtain a cosmetic product for make-up is particularly advantageous since all the problems of using loose dissolved before pressing are overcome.

In particular, the use of make-up elements made with make-up powder in agglomerated form allows a more precise and clean dosing on the bottom. As known, it is certainly easier to dose a granulated element, consisting of agglomerated make-up powder, than directly make-up powder.

Moreover, the make-up elements which are used for producing the cosmetic product may have a "prismatic" or cylindrical shape with a maximum diameter of between 2 and 4mm, preferably 2mm and a length preferably between 2 and 3 mm. Such dimensions are an excellent compromise between the manageability of the make-up elements and the filling capacity of the delimited volume before pressing.

Advantageously, the fact that the make-up elements are produced by pressing the cosmetic powder in the holes of a matrix, provides a very simple and quick system to agglomerate in an effective manner the cosmetic powder, which will be used in the subsequent steps, allowing the production of a granulated make-up powder. This is to all advantage of cleaning and ease of dosing within the productive chain of the cosmetic product for make-up 50.

Obviously, however, the agglomerated make-up powder granulate, made for example of the above-described composition, may be obtained with any other known method.

The above-described cosmetic product 50, as well as the make-up elements 1, have make-up features which depend on the type of cosmetic powder used to produce them, and can for example be blusher, blush, face powders, eye shadows, lighting products, colored or pearly cosmetic products in general, pressed and scented powders, etc.

One or more embodiments of the invention, which is defined in appended claims, may further include one or more of the following features, alone or in combination.

In particular, one embodiment relates to a method for producing a cosmetic product for make-up comprising the steps of:
a. preparing within a delimited volume a plurality of make-up elements consisting of an agglomerated make-up powder, and
b. compressing the make-up elements within the delimited volume with such a pressure as to crush and/or deform them so as to allow them to fully occupy the delimited volume.
According to one aspect, the make-up elements are shaped with a mainly axial development and are entirely made of pressed make-up powder. They have the same cross section for most of their length.
According to another aspect, the first base has a concave shape, while the second base has a convex shape.
According to a further aspect, the make-up elements arranged in the delimited volume are of at least two different colors.
According to yet another aspect, the delimited volume is defined at least at the bottom by the surface of a bottom and/or in which the delimited volume is defined both inferiorly and laterally by the surface of a bottom.
According to one aspect, the plurality of make-up elements is produced according to the following steps:
- mixing at least some pigments/beads, a filler and a binder to obtain a make-up powder
- pressing the make-up powder on a first surface of a matrix provided with a plurality of through holes, thus pushing the make-up powder through a first end of said holes to form a plurality of strips which protrude from a second end of the holes placed on a second surface of the matrix, and
- breaking the plurality of strips in the vicinity of said
According to another aspect, the strips are broken by a blade movable relative to the matrix which breaks them when they protrude from the second end of the holes by a length of between 2 and 4mm, preferably 2mm.
According to another aspect, the holes of the matrix have a maximum diameter of between 2 and 3mm.
According to a further aspect, the pressing occurs via at least one roll with an axis of rotation parallel and/or perpendicular to said first surface of the matrix.
According to yet another aspect, the make-up powder consists of at least one binder in a percentage from 5 to 10%, pigments/beads in a percentage from 5 to 43%, and fillers in a percentage from 13 to 90%, and optionally flow agents in a percentage from 0.5 to 1.5% and/or perfume in a percentage from 0.1 to 1% and/or preservatives in a percentage from 0.3 to 1%.
A cosmetic product for make-up produced according to the method of one or more of the preceding claims. A plurality of make-up elements according to one or more of the preceding claims, may be used in a product for make-up 18 comprising a container 10 containing said plurality of make-up elements. Said elements may be randomly arranged within the container and/or have at least two different colors.

## Claims

1. A method for producing make-up elements (1) comprising the steps of:
- mixing at least some pigments/beads, a filler and a binder to obtain a make-up powder (2) having a moisture between 0.8 and 1,5%
- pressing the make-up powder (2) on a first surface (23) of a matrix (20) having a plurality of through holes (21), thus pushing the make-up powder through a first end of said holes to form a plurality of strips (L) which protrude from a second end of the holes (21) disposed on a second surface (24) of the matrix (20), and
- breaking the plurality of strips (L) in the proximity of said second surface, so as to form a plurality of make-up elements (1).

2. A method according to the preceding claim, wherein the strips (L) are broken by a blade (30) movable relative to the matrix (20) which breaks them when they protrude from the second end of the holes (21) of a length between 2 and 15mm, preferably between 4 and 15mm, even more preferably 8mm.

3. A method according to one or more of the preceding claims, wherein the holes (21) of the matrix (20) have a maximum diameter between 2 and 10mm, preferably 6mm.

4. A method according to one or more of the preceding claims, wherein the pressing occurs via at least one roll (22, 22") with an axis of rotation parallel and/or perpendicular to said first surface (23) of the matrix.

5. A method according to one or more of the preceding claims, wherein the make-up powder is formed of at least one binder in a percentage from 5 to 10%, pigments/beads in a percentage from 5 to 43%, and fillers in a percentage from 13 to 90%, and optionally flow agents in a percentage from 0.5 to 1.5% and/or perfume in a percentage from 0.1 to 1% and/or preservatives in a percentage from 0.3 to 1%.

6. A make-up element (1) obtained through the method of claim 1, comprising a body entirely made of a pressed make-up powder, with a mainly axial development and a same cross section for most of its length, the body being provided with a lateral wall (4) with defined and regular surface and a first base (5) and a second base (6) with irregular surface.

7. A make-up element according to claim 6, wherein the first base (5) has concave shape, while the second base (6) has a convex shape.

8. A make-up element according to claim 6, wherein the body has a width between 2 and 10mm, preferably 6mm, and a length between 2 and 15mm, preferably between 4 and 15mm, even more preferably 8mm.

9. A make-up element (1) according to claim 6, wherein the body is multi-layered and has at least a first layer (9) of pressed make-up powder, axially superimposed on a second layer (8) of pressed make-up powder of different color.

10. A production method of a cosmetic make-up product (50) comprising the steps of:
a. disposing within a delimited volume (58) a plurality of make-up elements (1) obtained according to one or more of claims 1-5, and
b. pressing the make-up elements (1) within the delimited volume with a pressure sufficient to crush and/or deform them so as to allow them to fully occupy the delimited volume.

11. A method according to claim 10, wherein the delimited volume (58) is defined at least at the bottom by the surface of a bottom (51) and/or in which the delimited volume is defined both inferiorly and laterally by the surface of a bottom (51).

12. A method according to claim 10, wherein the compression step is carried out by means of a mold (52), preferably through the interposition of a canvas (53).

13. A method according to one or more of the preceding claims, wherein after compression step, the superficial part of the semi-finished product is removed.

14. A method according to the preceding claim, wherein after the removal of the superficial part, a further pressing is carried out with a smooth or sunken mold.

15. A cosmetic make-up product (50) produced according to the method of one or more of claims 10 to 14.

## Patentansprüche

1. Verfahren zum Erzeugen von Schminkelementen (1), das die Schritte umfasst:
- Mischen von mindestens einigen Pigmenten/Perlen, einem Füllstoff und einem Bindemittel, um ein Schminkpulver (2) mit einer Feuchtigkeit zwischen 0,8 und 1,5 % zu erhalten
- Pressen des Schminkpulvers (2) auf einer ersten Oberfläche (23) einer Matrix (20) mit einer Vielzahl von Durchgangslöchern (21), um auf diese Weise das Schminkpulver durch ein erstes Ende der Löcher zu drücken, um eine Vielzahl von Streifen (L) zu bilden, die aus einem zweiten Ende der Löcher (21) herausragen, die auf einer zweiten Oberfläche (24) der Matrix (20) angeordnet werden, und
- Brechen der Vielzahl von Streifen (L) in der Nähe der zweiten Oberfläche, um damit eine Vielzahl von Schminkelementen (1) zu bilden.

2. Verfahren nach dem vorhergehenden Anspruch, wobei die Streifen (L) durch eine bezogen auf die Matrix (20) bewegliche Klinge (30) gebrochen werden, die sie bricht, wenn sie aus dem zweiten Ende der Löcher (21) mit einer Länge zwischen 2 und 15 mm, vorzugsweise zwischen 4 und 15 mm, sogar noch bevorzugter 8mm herausragen.

3. Verfahren nach einem oder mehreren der vorhergehenden Ansprüche, wobei die Löcher (21) der Matrix (20) einen Höchstdurchmesser zwischen 2 und 10 mm, vorzugsweise 6 mm aufweisen.

4. Verfahren nach einem oder mehreren der vorhergehenden Ansprüche, wobei das Pressen mittels mindestens einer Walze (22, 22") mit einer zu der ersten Oberfläche (23) der Matrix parallelen und/oder senkrechten Drehachse entsteht.

5. Verfahren nach einem oder mehreren der vorhergehenden Ansprüche, wobei das Schminkpulver aus mindestens einem Bindemittel mit einem Anteil von 5 bis 10 %, Pigmenten/Perlen mit einem Anteil von 5 bis 43 % und Füllstoffen mit einem Anteil von 13 bis 90 % und wahlweise Fließmitteln mit einem Anteil von 0,5 bis 1,5 % und/oder Parfüm mit einem Anteil von 0,1 bis 1 % und/oder Konservierungsstoffen mit einem Anteil von 0,3 bis 1 % gebildet wird.

6. Schminkelement (1), das durch das Verfahren nach Anspruch 1 erhalten wird, umfassend einen vollständig aus einem gepressten Schminkpulver hergestellten Körper, mit einem hauptsächlich axialen Aufbau und einem gleichen Querschnitt für den größten Teil seiner Länge, wobei der Körper mit einer Seitenwand (4) mit definierter und regelmäßiger Oberfläche und einem ersten Boden (5) und einem zweiten Boden (6) mit unregelmäßiger Oberfläche bereitgestellt wird.

7. Schminkelement nach Anspruch 6, wobei der erste Boden (5) eine konkave Form aufweist, während der zweite Boden (6) eine konvexe Form aufweist.

8. Schminkelement nach Anspruch 6, wobei der Körper eine Breite zwischen 2 und 10 mm, vorzugsweise 6 mm, und eine Länge zwischen 2 und 15 mm, vorzugsweise zwischen 4 und 15 mm, sogar noch bevorzugter 8 mm aufweist.

9. Schminkelement (1) nach Anspruch 6, wobei der Körper mehrschichtig ist und mindestens eine erste Schicht (9) aus gepresstem Schminkpulver aufweist, die axial über eine zweite Schicht (8) aus gepresstem Schminkpulver unterschiedlicher Farbe gelegt wird.

10. Herstellungsverfahren eines Kosmetik-Schminkprodukts (50), das die Schritte umfasst:
a. Anordnen einer nach einem oder mehreren Ansprüchen 1 bis 5 erhaltene Vielzahl von Schminkelementen (1) innerhalb eines begrenzten Volumens (58), und
b. Pressen der Schminkelemente (1) in dem begrenzten Volumen mit einem Druck, der ausreicht, sie zu zerkleinern und/oder zu verformen, um ihnen zu ermöglichen, das begrenzte Volumen vollständig einzunehmen.

11. Verfahren nach Anspruch 10, wobei das begrenzte Volumen (58) mindestens an dem Boden durch die Oberfläche eines Bodens (51) definiert wird, und/oder in dem das begrenzte Volumen sowohl innen als auch seitlich durch die Oberfläche eines Bodens (51) definiert wird.

12. Verfahren nach Anspruch 10, wobei der Verdichtungsschritt mittels einer Form (52) durchgeführt wird, vorzugsweise durch die Einfügung eines Gewebes (53).

13. Verfahren nach einem oder mehreren der vorhergehenden Ansprüche, wobei nach dem Verdichtungsschritt der oberflächliche Bestandteil des Halbfertigprodukts entfernt wird.

14. Verfahren nach dem vorhergehenden Anspruch, wobei nach dem Entfernen des oberflächlichen Bestandteils ein weiteres Pressen mit einer glatten oder versenkten Form durchgeführt wird.

15. Kosmetik-Schminkprodukt (50), hergestellt nach dem Verfahren eines oder mehrerer Ansprüche 10 bis 14.

## Revendications

1. Procédé permettant la production d'éléments de maquillage (1) comprenant les étapes de :
- mélange d'au moins certains pigments/perles, d'une charge et d'un liant pour obtenir une poudre de maquillage (2) possédant une humidité comprise entre 0,8 et 1,5 %
- pressage de la poudre de maquillage (2) sur une première surface (23) d'une matrice (20) possédant une pluralité de trous traversants (21), poussant ainsi la poudre de maquillage à travers une première extrémité desdits trous pour former une pluralité de bandes (L) dépassant depuis une seconde extrémité des trous (21) disposés sur une seconde surface (24) de la matrice (20), et
- rupture de la pluralité de bandes (L) à proximité de ladite seconde surface, de façon à former une pluralité d'éléments de maquillage (1).

2. Procédé selon la revendication précédente, lesdites bandes (L) étant rompues par une lame (30) mobile par rapport à la matrice (20) les rompant lorsqu'elles dépassent depuis la seconde extrémité des trous (21) d'une longueur comprise entre 2 et 15 mm, de préférence comprise entre 4 et 15 mm, idéalement étant de 8 mm.

3. Procédé selon l'une quelconque ou plusieurs des revendications précédentes, lesdits trous (21) de la matrice (20) possédant un diamètre maximum compris entre 2 et 10 mm, de préférence de 6 mm.

4. Procédé selon l'une quelconque ou plusieurs des revendications précédentes, ledit pressurage se produisant par l'intermédiaire d'au moins un rouleau (22, 22") avec un axe de rotation parallèle et/ou perpendiculaire à ladite première surface (23) de la matrice.

5. Procédé selon l'une quelconque ou plusieurs des revendications précédentes, ladite poudre de maquillage étant formée d'au moins un liant selon un pourcentage allant de 5 et 10 %, de pigments/perles selon un pourcentage allant de 5 à 43 % et de charges selon un pourcentage allant de 13 à 90 % et éventuellement d'agents fluidifiants selon un pourcentage allant de 0,5 à 1,5 % et/ou de parfum selon un pourcentage allant de 0,1 à 1 % et/ou d'agents de conservation selon un pourcentage allant de 0,3 à 1 %.

6. Élément de maquillage (1) obtenu par le procédé selon la revendication 1, comprenant un corps entièrement constitué d'une poudre de maquillage pressée, avec un développement principalement axial et une même section transversale pour la majeure partie de sa longueur, ledit corps étant doté d'une paroi latérale (4) avec une surface définie et régulière et une première base (5) et une seconde base (6) avec une surface irrégulière.

7. Élément de maquillage selon la revendication 6, ladite première base (5) présentant une forme concave tandis que la seconde base (6) présente une forme convexe.

8. Élément de maquillage selon la revendication 6, ledit corps possédant une largeur comprise entre 2 et 10 mm, de préférence étant de 6 mm et une longueur comprise entre 2 et 15 mm, de préférence comprise entre 4 et 15 mm, idéalement étant de 8 mm.

9. Élément de maquillage (1) selon la revendication 6, ledit corps étant multicouche et possédant au moins une première couche (9) de poudre de maquillage pressée, superposée axialement sur une seconde couche (8) de poudre de maquillage pressée de couleur différente.

10. Procédé de fabrication d'un produit de maquillage cosmétique (50) comprenant les étapes de :
a. disposition dans un volume délimité (58) d'une pluralité d'éléments de maquillage (1) obtenus selon l'une quelconque ou plusieurs des revendications 1 à 5, et
b. pressage des éléments de maquillage (1) dans le volume délimité avec une pression suffisante pour les écraser et/ou les déformer de façon à leur permettre d'occuper entièrement le volume délimité.

11. Procédé selon la revendication 10, ledit volume délimité (58) étant défini au moins au niveau du fond par la surface d'un fond (51) et/ou ledit volume délimité étant défini intérieurement et latéralement par la surface d'un fond (51).

12. Procédé selon la revendication 10, ladite étape de compression étant effectuée au moyen d'un moule (52), de préférence par l'interposition d'une toile (53).

13. Procédé selon l'une quelconque ou plusieurs des revendications précédentes, après l'étape de compression, ladite partie superficielle du produit semi-fini étant retirée.

14. Procédé selon la revendication précédente, après le retrait de la partie superficielle, un pressage supplémentaire étant réalisé avec un moule lisse ou creux.

15. Produit de maquillage cosmétique (50) fabriqué selon le procédé de l'une ou de plusieurs des revendications 10 à 14.
